(19)

**Europäisches Patentamt**
**European Patent Office**
**Office européen des brevets**

(11) **EP 2 208 797 A2**

(12) **EUROPEAN PATENT APPLICATION**

(43) Date of publication:
**21.07.2010 Bulletin 2010/29**

(51) Int Cl.:
***C12Q 1/68*** (2006.01)

(21) Application number: **10000722.8**

(22) Date of filing: **01.03.2005**

(84) Designated Contracting States:
**AT BE BG CH CY CZ DE DK EE ES FI FR GB GR HU IE IS IT LI LT LU MC NL PL PT RO SE SI SK TR**

(30) Priority: **01.03.2004 US 549490 P**

(62) Document number(s) of the earlier application(s) in accordance with Art. 76 EPC:
**05724335.4 / 1 721 011**

(71) Applicant: **Applied Biosystems, LLC Carlsbad, CA 92008 (US)**

(72) Inventor: **Chen, Caifu Palo Alto, CA 94303 (US)**

(74) Representative: **Grund, Martin GRUND Intellectual Property Group Postfach 44 05 16 80754 München (DE)**

Remarks:
This application was filed on 25-01-2010 as a divisional application to the application mentioned under INID code 62.

(54) **Methods, compositions and kits for use in polynucleotide amplification**

(57) In some embodiments, the present disclosure provides admixtures and methods for PCR amplification of a target polynucleotide in the presence of forward and reverse amplification primers and in which the occurrence of primer-dimers is decreased by including a dummy target polynucleotide in the PCR reaction mix. The dummy target polynucleotide is simultaneously amplified by the amplification primers and is designed to form a less stable hybrid with an amplification primer as compared to the hybrid formed between the amplification primer and the target polynucleotide. Kits for carrying out the various methods are also provided.

EP 2 208 797 A2

## Description

### FIELD

The present disclosure relates to the field of molecular biology, and more specifically, relates to methods, compositions and kits for carrying out polynucleotide amplification reactions.

### BACKGROUND

The technology of PCR permits amplification and subsequent detection of minute quantities of a target nucleic acid. Details of PCR are well described in the art, including, for example, U.S. Patent Nos. 4,683,195 to Mullis et al., 4,683,202 to Mullis and 4,965,188 to Mullis et al. Generally, amplification primers are annealed to the denatured strands of a target nucleic acid, and primer extension products are formed by the polymerization of deoxynucleoside triphosphates by a polymerase. A typical PCR method involves repetitive cycles of template nucleic acid denaturation, primer annealing and extension of the annealed primers by the action of a thermostable polymerase. The process results in exponential amplification of the target nucleic acid, and thus allows the detection of targets existing in very low concentrations in a sample.

PCR is widely used in a variety of applications, including biotechnological research, clinical diagnostics and forensics. However, the methodology is subject to practical limitations that result in less than optimal efficiency and specificity. In particular, before the first cycle of a PCR experiment (i.e. at "zero cycle"), the reagents for amplification are typically mixed and stored at room temperature or lower. Because thermostable polymerases, for example, Thermus aquaticus (Taq) polymerase, have residual activity even at 0°C, relatively large quantities of non-specific products can be formed by low stringency priming and replication. The non-specific products, known as zero cycle artifacts, include primer-dimers formed by ligation of primers having homology at their 3' ends. Because of the micromolar concentrations of primers used in PCR relative to the often minute concentrations of target, the formation of primer-dimers can significantly interfere with the efficiency of target amplification. Primer-dimers are thus particularly pervasive zero-cycle artifacts.

### SUMMARY

In one aspect, the disclosure concerns methods of amplifying a polynucleotide sequence of interest. In some embodiments, the method comprises amplifying a target polynucleotide in the presence of a polymerase, a forward amplification primer, a reverse amplification primer, dNTPs suitable for template-dependent primer extension, and a dummy target polynucleotide. The target polynucleotide comprises (i) a first target strand comprising a 5'-region, the complement of which is capable of hybridizing to the forward amplification primer; a 3'-region capable of hybridizing to the reverse amplification primer; a target sequence of interest disposed between the 5'- and 3'-regions; and (ii) an optional second target strand that is complementary to the first target strand. The dummy target polynucleotide comprises (i) a first dummy strand comprising a 5'-region, the complement of which is capable of hybridizing to the forward amplification primer; a 3'-region capable of hybridizing to the reverse amplification primer; an optional intermediate region disposed between the 5' and 3'-regions; and (ii) an optional second dummy strand complementary to the first dummy strand. The dummy target polynucleotide is designed such that hybrids that form between the dummy target polynucleotide and one or both of the amplification primers are less stable as compared to the analogous hybrids that form between the target polynucleotide and the amplification primers. For example, in one embodiment, the sequence of the dummy target polynucleotide is designed such that a hybrid formed between the first dummy strand and the reverse amplification primer comprises at least one mismatch.

In other aspects, the disclosure provides compositions and kits for carrying out the methods. Exemplary kits comprise a dummy target polynucleotide, as described herein, and can comprise primers, enzymes and reagents for carrying out an amplification reaction. Kits can also comprise reagents for detecting the amplification products.

It has been surprisingly discovered in accordance with the present disclosure that amplification artifacts, such as primer-dimers, are reduced when a target polynucleotide is co-amplified in the presence of a dummy target polynucleotide, as described herein. The methods, compositions, and kits of the present disclosure facilitate reduction in amplification artifacts.

### BRIEF DESCRIPTION OF THE DRAWINGS

FIG. 1 is a schematic illustration of a target polynucleotide, a dummy target polynucleotide and forward and reverse primers.

FIG. 2 is an illustration of a polyacrylamide gel showing the results of the experiment in Example 1.

FIG. 3 is an illustration of a polyacrylamide gel showing the results of the experiment in Example 1.

FIG. 4 is an illustration of a polyacrylamide gel showing the results of the experiment in Example 1.

**DETAILED DESCRIPTION**

**1.1 Definitions**

In the present disclosure, "nucleoside" refers to a compound containing a base-pairing moiety (also referred to as a "base") such as a purine, deazapurine, or pyrimidine nucleoside base, e.g., adenine, guanine, cytosine, uracil, thymine, deazaadenine, deazaguanosine, inosine, or any functional equivalent thereof, which is attached to a backbone moiety such as a sugar ring or any functional equivalent thereof. Nucleosides include naturally occurring nucleosides which contain a base-pairing moiety (A, C, G, T, I or U) linked to the 1-carbon of a pentose ring, 2'-deoxy and 2'-hydroxyl forms thereof, and also pentose analogs and ring-open equivalents thereof. The term "nucleobase" comprises those naturally occurring and those non-naturally occurring heterocyclic moieties commonly known to those who utilize nucleic acid technology or utilize peptide nucleic acid technology to thereby generate polymers which can sequence specifically bind to nucleic acids.

The term "nucleotide" as used herein refers to a phosphate ester of a nucleoside, e.g., a triphosphate ester, wherein the most common site of esterification is the pentose 5'-hydroxyl group. In certain cases, term "nucleoside" refers both nucleosides and nucleotides, for convenience. The terms nucleotide and nucleoside as used herein are intended to include synthetic analogs having modified nucleoside base moieties, modified sugar moieties, and/or modified phosphate groups and phosphate ester moieties, e.g., as described elsewhere.

"Polynucleotide" refers to a polymer of nucleoside monomers, including single, double and triple stranded deoxyribonucleotides, ribonucleotides, $\alpha$-anomeric forms thereof, and the like. Usually the nucleoside monomers are linked by phosphodiester linkages, such that "phosphodiester linkage" refers to a phosphate ester bond or analog thereof wherein the phosphorous atom is in the +5 formal oxidation state and one or more of the oxygen atoms is replaced with a non-oxygen moiety. In some embodiments, polynucleotides comprise phosphate analogs such as phosphorothioate, phosphorodithioate, phosphoroselenoate, phosphorodiselenoate, phosphoroanilothioate, phosphoranilidate, phosphoramidate, boronophosphates, and the like, including associated counterions, e.g., $H^+$, $NH_4^+$, $Na^+$, and the like, if such counterions are present. In some embodiments, polynucleotides comprise polymers of non-nucleotidic monomers, linked by phosphate ester or other linkages, which are capable of forming sequence-specific hybrids with a target nucleic acid, e.g., peptide nucleic acids (PNAs). Chimeric structures containing more than one type of linkage and/or nucleotide subunit are also contemplated. Polynucleotides typically range in size from a few monomeric units, e.g. 8-40, to hundreds or thousands of monomeric units. Whenever a polynucleotide is represented by a sequence of letters, such as "ATGCCTG," it will be understood that the nucleotides are in 5' to 3' order from left to right and that "A" denotes deoxyadenosine, "C" denotes deoxycytidine, "G" denotes deoxyguanosine, "1" denotes inosine, "U" denotes uracil, and "T" denotes thymidine.

As used herein, the term "primer" refers to a polynucleotide that is capable of hybridizing (i.e. annealing) with a polynucleotide and serving as an initiation site for nucleotide polymerization.

"Primer extension" is the enzymatic addition, i.e. polymerization, of monomeric nucleotide units to a primer while the primer is hybridized (annealed) to a template polynucleotide. Primer extension is initiated at the template site where a primer anneals.

The term "primer dimer" refers to template-independent non-specific amplification product, which is believed to result from primer extensions wherein another primer serves as a template. Although primer dimer frequently appears to be a concatamer of two primers, i.e., a dimer, concatamers of more than two primers also occur. The term "primer dimer" is used herein generically to encompass template-independent non-specific amplification product.

An "amplicon" is a polynucleotide product generated in an amplification reaction.

A "target polynucleoitde" has a target sequence of interest to be replicated, and may be either single-stranded or double stranded, and may include sequences in addition to the target sequence, which additional sequences may not be amplified.

An "amplification reaction" is a template-dependent in vitro enzyme-catalyzed reaction for increasing the number of target polynucleotides.

**1.2 Methods, Compositions and Kits**

The principles of PCR and the conditions for amplification and detection of target polynucleotides are well known in the art and may be found in numerous references known to the skilled artisan, including, for example, U.S. Patent Nos. 4,683,195, 4,683,202, and 4,965,188. Briefly, a sample containing, or suspected of containing, a target polynucleotide is heated to denature double-stranded polynucleotides in the presence of a forward and a reverse amplification primer that are complementary to sequences flanking a target sequence of interest to be amplified. The primers anneal to the separated target strands and are extended from each 3' hydroxyl end by a polymerizing agent such as a thermostable polymerase. Double-stranded or single-stranded DNA can be amplified by PCR. RNA can also serve as a target by reverse transcribing RNA into cDNA. The steps of denaturation, primer annealing and DNA synthesis are carried out at discrete temperatures, and repeated cycles result in exponential accumulation of the target nucleic acid. The PCR vessel can be a stoppered plastic vessel, a cuvette, well, or pouch as described in U.S. Patent No. 5,229,297. Reagents for PCR amplification are typically mixed in a single vessel, and generally include primers, nucleoside triphosphates (generally dATP, dCTP, dGTP and dTTP or dUTP), thermostable DNA polymerase, magnesium containing buffer, and target polynucleotide. Reagents and conditions for PCR are well-known to one of ordinary skill in the art (see, e.g., Sambrook, et al., 1989, Molecular Cloning--A Laboratory Manual, Cold Spring Harbor Laboratory, Cold Spring Harbor and Ausubel, et al., 1995, Current Protocols in Molecular Biology, Chapter 15, John Wiley & Sons). For amplification of RNA targets, a reverse transcriptase may be utilized in addition to or in lieu of the thermostable DNA polymerase. Thermostable reverse transcriptase are particularly useful, as are thermostable DNA polymerases having reverse transcriptase activity. Methods for PCR amplification of RNA targets are known to one of ordinary skill in the art and described, for example, in U.S. Patent Nos. 5,176,995, 5,310,652 and 5,322,770. Sample preparation methods suitable for amplification reactions are well known in the art and fully described in the literature cited herein. The particular method used is not a critical for success. One of skill in the art can optimize reaction conditions for use with the known sample preparation methods. As will be appreciated by skilled artisans, target polynucleotides suitable for amplification may be either DNA (e.g., cDNA or genomic DNA) or RNA (e.g., mRNA or rRNA) in nature, and may be derived or obtained from virtually any sample or source, wherein the sample may, optionally, be scarce or of a limited quantity. For example, the sample may be one or a few cells collected from a crime scene or a small amount of tissue collected via biopsy.

In some embodiments, a target polynucleotide is the product of an oligonucleotide ligase assay (OLA) (see, e.g., U.S. Patent Nos. 6,297,016, 6,025,139). OLA typically utilize at least two oligonucleotide probes which hybridize adjacently to a genomic template polynucleotide, and which are subjected to ligation. The OLA is a "yes-no" test used to determine whether a particular base is present or not at a predetermined position in a genomic template. Applicants found that when the products of an OLA reaction were subjected to PCR amplification, residual amounts of the oligonucleotide probes apparently nonspecifically hybridized with each other and/or with the PCR amplification primers, resulting in various amplification artifacts. Control ligation reactions were performed in the absence of genomic template (i.e., "no template control" reactions), and the PCR amplification of such control reactions generally resulted in significant levels of amplification artifacts. This high background made the OLA difficult to interpret. The presence of a dummy target polynucleotide during PCR of the OLA product, and during the PCR of the products of the "no template control," was found to markedly reduce the background by largely eliminating these amplification artifacts (see Example 1 hereinbelow).

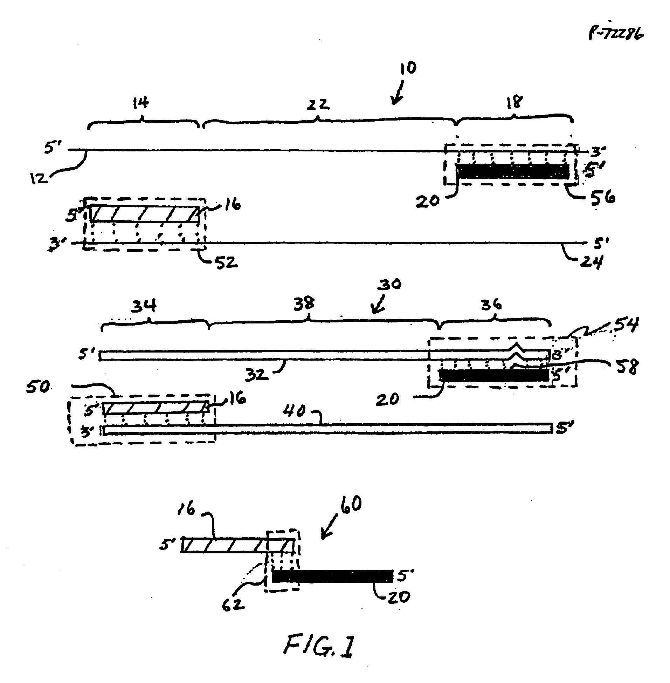

The present disclosure provides a modification of known methods of PCR in order minimize the formation of artifacts during amplification of at least a portion of a polynucleotide. In one aspect the present disclosure concerns methods of amplifying a sequence of interest in a target polynucleotide. In some embodiments, the amplification comprises a conventional PCR amplification with the exception that it also includes a dummy target polynucleotide, as described herein, in the reaction. Components present in some embodiments of an amplification reaction are illustrated in FIG. 1. In some embodiments, the methods comprise amplifying a target sequence **22** of a target polynucleotide **10** in the presence of a polymerase (not shown), a forward amplification primer **16,** a reverse amplification primer **20,** dNTPs suitable for template-dependent primer extension (not shown), and a dummy target polynucleotide **30**. The target polynucleotide comprises a first target strand **12**. The first target strand comprises a 5'-region **14,** the complement of which is capable of hybridizing to the forward amplification primer. Strand **12** also comprises a 3'region **18** capable of hybridizing to the reverse amplification primer **20,** and a sequence of interest **22** disposed between the 5'- and 3'-regions. The target polynucleotide can also include a second strand **24** that is complementary to the first strand **12**. Designation of the two strands of a duplex target polynucleotide as a "first target strand" and as a "second target strand" may be arbitrary, so that reversal of these designations may also be appropriate.

Also included in the reaction is a dummy target polynucleotide **30**. In some embodiments, the dummy target polynucleotide comprises a first dummy strand **32** which comprises a 5'-region **34,** the complement of which is capable of hybridizing to the forward amplification primer **16** and a 3'-region **36** which is capable of hybridizing to the reverse amplification primer **20**. An intermediate region **38** is disposed between the region **34** and region **36**.

The target polynucleotide and the dummy target polynucleotide have sequences that are capable of hybridizing with the amplification primers. Specifically, the forward amplification primer is capable of forming a hybrid **50** with optional second dummy strand **40,** and is also capable of forming a hybrid **52** with optional second target strand **24**. The reverse amplification primer **20** is capable of forming a hybrid **54** with the first dummy strand **32,** and also of forming a hybrid **56** with first target strand **12**.

While the dummy target polynucleotide can base pair with the primers, in some embodiments, its sequence is adjusted as described herein, so that the dummy:primer hybrids are less stable than the target:primer hybrids. The target sequence of interest is still capable of being amplified because the dummy:primer hybrids are less stable than the target:

primer hybrids. Applicants have discovered that the presence of the dummy target polynucleotide can lower, and/or essentially eliminate, the amount of primer dimer formed. FIG. 1 illustrates overlap **62** between the forward and reverse primers due to homology at their 3' ends, which can lead to primer dimer formation. Without wishing to be bound by theory, it is believed that the dummy:primer hybrids, such as shown at **50** and **52,** are more stable than hybrids that can form between primers, such as shown at **60,** and this difference in stability lessens the opportunity for primers to anneal to each other, and hence lowers primer dimer formation. It is believed that a suitable dummy target polynucleotide is amplified in the first and/or second round of amplification at an efficiency that is less than that of the target polynucleotide sequence. In some embodiments, the efficiency is about $10^2$-fold to about $10^6$-fold less than that of the target polynucleotide sequence. This lower efficiency can be accomplished by incorporating various features, as indicated below.

The stability of any of the hybrids **50, 52, 54** or **56** can be characterized by a free energy parameter, such as $\Delta G$ or $T_m$. The value of the free energy parameter can be determined, e.g., empirically, semi-empirically, or by calculation (e.g., by a calculation method described herein) or by any method known to those in the art. For the purpose of discussion, and not by way of limitation, all free energy parameter values referenced herein are calculated values.

$\Delta G$ refers to the free energy for a duplex. In some embodiments, this value is calculated by the nearest-neighbor method for DNA (SantaLucia, 1998, Proc. Natl. Acad. Sci. USA 95:1460-1465) and for RNA (Frier et al., 1986, Proc. Natl. Acad. Sci. USA 83:9373-9377). The free energy is calculated by the formula $\Delta G = \Delta H - T\Delta S$, wherein H is the enthalpy, S is the entropy and T is temperature. The free energy is a measure of stability, the greater the negative value, the more stable the duplex formed by the polynucleotide.

As shown in FIG. 1, the reverse amplification primer can form a hybrid **54** or a hybrid **56.** The forward amplification primer can form a hybrid **50** or **52.** As described hereinbelow, the sequences of regions **34** and/or **36** can be designed such that hybrids that form at these regions have selected $\Delta G$ values or selected $T_m$ values. In some embodiments, one or both of hybrids **50** and **54** have lower stability as compared to hybrids **52** and **56,** respectively.

In some embodiments, the $\Delta G$ of hybrid **50** is selected to be greater than the $\Delta G$ of hybrid **52** and/or the $\Delta G$ of hybrid **54** is selected to be greater than the $\Delta G$ of hybrid **56.** In some embodiments, the $\Delta G$ of hybrid **50** is selected to be at least about 1.5 kcal/mol greater than the $\Delta G$ of hybrid **52.** In some embodiments, the $\Delta G$ of hybrid **54** is selected to be at least about 1.5 kcal/mol greater than the $\Delta G$ of hybrid **56.** In some embodiments, the $\Delta G$ of hybrid **50** is selected to be at least about 1.5 kcal/mol greater than the $\Delta G$ of hybrid **52** and the $\Delta G$ of hybrid **54** is selected to be at least about 1.5 kcal/mol greater than the $\Delta G$ of hybrid **56.**

In some embodiments, the $\Delta G$ of hybrid 54 is selected to be in the range of about 1.5 - 10 kcal/mol greater than the $\Delta G$ of hybrid **56** and/or the $\Delta G$ of hybrid **54** is selected to be in the range of about 1.5 - 10 kcal/mol greater than the $\Delta G$ of hybrid **56.**

The stability of hybrids can be characterized by $T_m$. $T_m$ refers to the melting temperature (temperature at which 50% of a hybrid is a duplex). The greater the $T_m$ value, the more stable the hybrid. In some embodiments, the $T_m$ values are calculated using known methods for predicting oligonucleotide melting temperatures (see, e.g., SantaLucia, 1998, Proc. Natl. Acad. Sci. USA 95:1460-1465; Frier et al., 1986, Proc. Natl. Acad. Sci. USA 83:9373-9377; Breslauer, 1986, Proc. Natl. Acad, Sci. USA 83:3746-3750; Rychlik et al., 1989, Nucleic Acids Res. 17:8543-8551; Rychlik et al., 1989, Nucleic Acids Res. 18:6409-6412; Wetmur, 1991, Crit. Rev. Biochem. Mol. Biol. 26:227-259; Osborne, 1991, CABIOS 8:83; Montpetit et al., 1992, J. Virol. Methods 36:119-128).

In some embodiments, the $T_m$ of hybrid **52** is selected to be greater than the $T_m$ of hybrid **50** and/or the $T_m$ of hybrid **56** is selected to be greater than the $T_m$ of hybrid **54.** In some embodiments, the $T_m$ of hybrid **52** is selected to be at least about 2°C greater than the $T_m$ of hybrid **50.** In some embodiments, the $T_m$ of hybrid **56** is selected to be at least about 2°C greater than the $T_m$ of hybrid **54. In** some embodiments, the $T_m$ of hybrid **52** is selected to be at least about 2°C greater than the $T_m$ of hybrid **50** and/or the $T_m$ of hybrid **56** is selected to be at least about 2°C greater than the $T_m$ of hybrid **54.** In some embodiments, the $T_m$ of hybrid **52** is selected to be at least about 1 °C; 2°C, 3°C, 4°C, 5°C, 6°C, 7°C, 8°C, 9 °C, or 10°C greater than the $T_m$ of hybrid **50** and/or the $T_m$ of hybrid **56** is selected to be at least about 1 °C, 2°C, 3°C, 4°C, 5°C, 6°C, 7°C, 8°C, 9 °C, or 10°C greater than the $T_m$ of hybrid **54.**

The chemical composition of the dummy target polynucleotide is not critical, as long as it can function in the methods described herein. The sequence of a dummy strand can be designed and/or modified in any of variety of ways in order to reduce the ability of a primer to hybridize with a primer binding region on the dummy strand. In some embodiments, the reduction in hybridization can be the result of kinetic effects, thermodynamic effects, or both. A desired value for the stability of a hybrid (e.g., a $T_m$ or a $\Delta G$ value) can be achieved in any of a variety of different ways. In some embodiments, a hybrid can be destabilized, leading to a lower Tm (or a higher $\Delta G$ value), by including at least one mismatch within the hybrid. Non-limiting examples of a mismatch include insertion, deletion or substitution of one or more nucleotide. A substitution causing formation of a non-Watson Crick base pair is a specific example of a substitution. A mismatch can also result from the positioning of a modified nucleobase within a region, such as region **34** and/or region **36,** capable of hybridizing with a primer. Non-limiting examples of a modified nucleobase include 2'-deoxyisoinosine, 7-deaza-2'-deoxyinsosine, nitroazole and 5-nitroindole. A dummy target polynucleotide can consist solely of DNA or RNA. A dummy target polynucleotide can be a chimera that comprises regions of DNA, RNA, and/or LNA. For example, a dummy target polynucleotide can be a DNA/RNA chimera or an LNA/DNA chimera. In some embodiments, a dummy target polynucleotide is a LNA/DNA chimera and is comprised of: (i) a contiguous moiety of LNA monomer units and (ii) a contiguous moiety of nucleotide monomer units with an enzymatically-extendable 3'-hydroxyl terminus.

In some embodiments, the ability of a dummy strand is designed to include extra bases extending from the 3' and/or the 5' end which can form intramolecular secondary structure. An example of such a structure is a hairpin loop formed by such extra terminal bases which pair with other bases within a dummy strand (e.g., within a binding region).

The sequence of a dummy target polynucleotide can be designed such that at least one mismatch is positioned within either one or both of hybrids **54** and **50**. An example of such a mismatch is schematically illustrated by mismatch **58** in hybrid **54** FIG. 1. Hybrid **54** can comprise 1, 2, 3, 4, 5, 6, 7, 8 or more mismatches, for example.

A deletion need not occur in an internal site within a primer binding region. In some embodiments, a dummy strand is designed with a shortened, or truncated, primer binding region in which the dummy strand is designed to be short at its 5' and/or 3' terminus such that one or both primer binding regions have fewer bases than the respective amplification primer.

A dummy target polynucleotide can be any suitable length. In some embodiments, the length of a dummy target polynucleotide can be in the range of about 18 nucleotides to about 1000 nucleotides, in the range of about 20 nucleotides to about 500 nucleotides, or in the range of about 30 to about 200 nucleotides. In some embodiments, the length of the dummy target polynucleotide is in the range of about ½ to about 1.5 times the length of a target polynucleotide. The length of the region **34** and region **36** can be the same or can be different. In some embodiments, the length of these regions can be in the range of about 9 to about 50 nucleotides. In some embodiments, the length of the intermediate region **38** is in the range of about 0 to about 1000 nucleotides or in the range of about 25 to about 100 nucleotides. The intermediate region can consist of homopolymer (e.g. poly dT), or can include one or more regions of homopolymer. In some embodiments, the intermediate region contains A, C, G, T, U, and I in any sequence. In some embodiments, the sequence is designed to minimize interference with the amplification reaction. In some embodiments, the intermediate region is a unique sequence that is not recognized by amplification primers present in the reaction.

A mismatch can be located at any position within an amplification primer:dummy strand hybrid as long as the primer is still capable of being extended. For example, a mismatch can be located at approximately the center of the amplification primer:dummy strand hybrid. In another example, a mismatch can be located within the 3'-terminal half of the primer binding region of a dummy strand in an amplification primer:dummy strand hybrid. In some embodiments, the mismatch is positioned within about 20 nucleotides, within about 15 nucleotides, within about 10 nucleotides, within about 8 nucleotides, or within about 5 nucleotides from the 3' end of a dummy strand.

The amount of amplification primer hybridized to a dummy target polynucleotide will depend on the stability of the hybrid formed, the reaction conditions, and will also depend upon the relative concentration of the dummy target polynucleotide in the reaction mixture. For example, at a higher concentration of dummy target polynucleotide, and at a fixed concentration of target polynucleotide, more primer will be bound to the dummy target polynucleotide. Concentrations of primers will typically be those used in conventional PCR. For example, the concentration of forward and reverse primers can be in the range of about 5 nM to about 2,000 nM in an amplification. In another example, the concentrations of forward and reverse primers can be in the range of about 250 nM to about 1,000 nM in an amplification.

In an amplification reaction, the level of amplification products, including the level of primer-dimer, can be determined using a variety of methods. For example, the amplification products can be separated by any suitable size-dependent process, e.g., gel electrophoresis, capillary electrophoresis or chromatography; and the separated fragments detected, e.g., by laser-induced fluorescence. In some embodiments, the product of the amplification reaction is analyzed at a selected end-point (e.g., in the exponential phase, the linear phase, or in the plateau phase of the amplification) by use of conventional slab gel electrophoresis and visualized using conventional stains such as ethidium bromide, dye (e.g., SYBR Green I) or silver stain. The amplification products can also be quantified using conventional real-time methods.

In an amplification, the concentration of polymerase, dNTPs, amplification primers and other reagents are not critical as long as they are present in sufficient amount to function adequately in the method. Concentrations used in conventional PCR reactions may be used. For example, in some embodiments, the polymerase(s) is(are) present in the incubation in the can range of about 0.1 units/μl to about 10 units/μl. Suitable concentrations for the amplification primer (s) can be determined by performing reactions at various concentrations of these primers. In some embodiments, the concentration of each amplification primer in the reaction mixture can range from about 5nM to about 4 μM and these primers are present in equimolar concentrations. The concentration of each deoxyribonucleotide triphosphate can be in the range of about 50 μM to about 2 mM at the start of the amplification. The target polynucleotide can be present at any suitable concentration, for example in the range of about 0.01 μg/μl - 10μg/μl. Suitable concentrations of a dummy target polynucleotide for use in an amplification reaction can be determined empirically, for example by titration.

For a selected dummy target polynucleotide the concentration of the dummy target polynucleotide can be selected relative to the concentration of target polynucleotide so as to minimize the formation of primer-dimers. In some embodiments, the initial molar ratio of target polynucleotide to the dummy target polynucleotide is greater than about 1.

In some embodiments, the ratio is greater than about 10. In some embodiments, the ratio is in the range of about 10 to about $10^3$, in the range of about 100 to about $10^4$, in the range of about 1 to about $10^6$, or in the range of about I to about $10^{10}$.

In an illustrative example, a set of amplification primers can be designed to amplify a target double-stranded target polynucleotide (having a first and a second strand) in a conventional PCR. The $T_m$ of a hybrid **56** formed between the reverse amplification primer and the first target polynucleotide strand can be calculated, as described above. A single-stranded dummy target polynucleotide can be designed having an intermediate region **38** of 25 nucleotides (poly-T), and having terminal regions **34** and **36** (each 20bp in length) designed such that the hybrid **54** comprises a single mismatch (e.g., a single G-T mismatch) located at 8 bases from the 3' terminus. The presence of the mismatch lowers the Tm of the hybrid **54** by about 5°C as compared to hybrid **56.** Amplification reactions can be conducted in the absence of the dummy target polynucleotide, and at a series of initial molar ratios of target polynucleotide to dummy target polynucleotide (e.g., 1, 10, 100, $10^3$, $10^4$, $10^5$, $10^6$, and $10^7$). The amplification can be allowed to proceed to the plateau phase (e.g., about 10 cycles). The amplification products, including those formed from the target polynucleotide, the dummy target polynucleotide, and any amplification artifacts such as primer-dimers, can be analyzed using gel electrophoresis followed by SYBR green I staining. The molar ratio at which primer-dimer formation is essentially undetectable (e.g., by use of visual inspection and/or by gel scanning) can be determined. For example, primer-dimers may be essentially undetectable a molar ratio of $10^4$, or at ratios below this value, but significant primer dimer formation may be observed at higher molar ratios. Other values of molar ratio can be tested above and below the value of $10^4$ in order to determine a preferred molar ratio. Various other dummy target polynucleotides (e.g., having additional sites of mismatch, other types of mismatch, and/or differently positioned mismatches) can be designed and similarly tested.

Amplification of the dummy target polynucleotide may compete with amplification of the target polynucleotide. The sequence of the dummy target polynucleotide, and/or its concentration, can be varied in order to minimize the formation of primer-dimers and also to minimize the effect on the amplification of target polynucleotide. In some embodiments, the dummy target polynucleotide has a sequence, and is present at a concentration, such that the level of primer-dimer at a selected end-point (e.g., at the exponential phase or at the plateau phase) of an amplification reaction, is less than about 10% of the level of the amplification product of the target sequence. In some embodiments, the level is less than about 1%, less than about 0.1% and less than about 0.01% of the level of the amplification product of the target sequence. In some embodiments, the dummy target polynucleotide has a sequence, and is present at a concentration, such that primer-dimer is essentially undetectable using a conventional staining method as described above. In some embodiments, the dummy target polynucleotide has a sequence, and is present at a concentration, such that after about 20 amplification cycles, the amount of target polynucleotide amplicon generated is approximately 10-fold greater than the amount of dummy target polynucleotide amplicon generated.

In some embodiments, the dummy target polynucleotide has a sequence, and is present at a concentration, such that the level of primer-dimer at a selected end-point (e.g., at the exponential phase or at the plateau phase) of a background control amplification reaction carried out in the absence of target polynucleotide, but in the presence of dummy target polynucleotide, is less than about 10% of the level of primer-dimer formed in a similar reaction carried out in the absence of dummy target polynucleotide. In some embodiments, the level of primer-dimer in the presence of the dummy target polynucleotide is less than about 1%, less than about 0.1%, less than about 0.01% and less than about 0.001% of the level formed in the absence of the dummy target polynucleotide.

In some embodiments, one or both of the amplification primers **16** and **20** used in the method are universal primers. The term "universal primer" refers to an amplification primer comprising a universal sequence. The term "universal sequence" refers to a sequence contained in a plurality of primers, but preferably not in a complement to the sequence of interest in the target polynucleotide (e.g., region **22),** such that a primer composed entirely of universal sequence is not capable of hybridizing with the sequence of interest. The term "universal sequence" also refers to a sequence contained in a plurality of primers, but preferably not in a complement to the intermediate region of a dummy target polynucleotide (e.g., region **38),** such that a primer composed entirely of universal sequence is not capable of hybridizing with the intermediate region of a dummy target polynucleotide. In some embodiments, the universal forward amplification priming sequence and the universal reverse amplification priming sequence are selected from a randomized pool of sequences unique to the human genome.

In some embodiments, the dummy target polynucleotide is single stranded and has the following formula:

$$UP_1\text{-}(B)_n\text{-}UP_2$$

wherein $UP_1$ represents a sequence, the complement of which is capable of hybridizing to a universal priming sequence; $UP_2$ represents a sequence capable of hybridizing to a universal priming sequence; each B, independently of the others, represents a nucleotide selected from A, C, T, G, I and U; and n represents an integer from 0 to 1000. The sequence

of UP$_1$ is such that a hybrid formed between the complement of UP$_1$ and a universal priming sequence comprises at least one mismatch and/or the sequence of UP$_2$ is such that a hybrid formed between UP$_2$ and a universal priming sequence comprises at least one mismatch. Specific examples of dummy target polynucleotides are identified as SEQ ID Nos. 4 - 7 in the Examples herein.

According to some embodiments of the instant disclosure, an amplification primer pair comprises two amplification primers, one forward amplification primer and one reverse amplification primer, as is well-known in the art. The amplification primer pair may be sequence-specific and may be designed to hybridize to sequences that flank a sequence of interest to be amplified. Thus, the actual nucleotide sequences of a primer pair may depend upon the sequence of interest to be amplified, and will be apparent to those of skill in the art. Methods for designing primer pairs suitable for amplifying specific sequences of interest *via* PCR or RT-PCR are well-known. *See e.g.*, Eckert et al. (1991) PCR: A Practical Approach, McPherson, Quirke, and Taylor eds., IRL Press, Oxford, Vol. 1, pp. 225-244;. TaqMan® Universal PCR Master Mix Protocol (available from Applied Biosystems, an Applera Corporation business, Cat. # 4304449 Rev. C); Rozen et al., 2000, Bioinformatics Methods and Protocols: Methods in Molecular Biology, Humana Press, Totowa, NJ, pp 365-386; www.ucl.ac.uk/wibr/2/services/reldocs/taqmanpr.pdf; www.ukl.uni-freiburg.de/core-facility/taqman/taqindex.html; www.operon.com/oligos/toolkit.php; www-genome.wi.mit.edu/cgi-bin/primer/primer3_www.cgi; www.ncbi.nlm.nih.gov/BLAST/; and www.biotech.uiuc.edu/primer.htm, which provide examples demonstrating how particular primer pairs may be designed.

Depending upon the nature of the sample polynucleotides to be amplified (e.g., RNA or DNA), an amplification reaction can be accomplished by polymerase chain reaction (PCR) or reverse-transcription PCR (RT-PCR). Thus, amplifications in which the target polynucleotide(s) is a DNA will typically include as essential components, in addition to amplification primers or sets discussed above, a mixture of 2'-deoxyribonucleoside triphosphates suitable for template-dependent DNA synthesis (e.g., primer extension) and a DNA polymerase. As mentioned above, with the exception of the dummy target polynucleotide, the amplification reactions may be carried out using reagents, reagent concentrations and reaction conditions conventionally employed in conventional PCR and RT-PCR reactions. For example, the various different primer concentrations, enzymes (*e.g.* DNA polymerases and reverse transcriptases), enzyme concentrations, dNTP mixtures (as well as their absolute and/or relative concentrations), total target polynucleotide concentrations, buffers, buffer concentrations, pH ranges, cycling times and cycling temperatures employed in conventional PCR and RT-PCR reactions may be used for the amplification reactions described herein. The reaction will include a suitable buffer (e.g., pH 7-8) which will typically contains Mg$^{2+}$. Guidance for selecting suitable reaction conditions may be found, for example, in U.S. Patent Nos. 4,683,202; 4,683,195; 4,800,159; 4,965,188; 5,561,058; 5,618,703; 5,693,517; 5,876,978; 6,087,098; 6,436,677; and 6,485,917, and PCR Essential Data, J. W. Wiley & Sons, Ed. C.R. Newton, 1995, and PCR Protocols: A Guide to Methods and Applications. (Innis, M, Gelfand, D., Sninsky, J. and White, T., eds.), Academic Press, San Diego (1990), all of which are incorporated herein by reference. A variety of tools for designing PCR and RT-PCR amplification primers, as well as myriad protocols, reaction conditions and techniques for carrying out various different types of PCR reactions, including conventional PCR reactions and RT-PCR reactions are also available online (*see, e.g.*, protocol-online.org/prot/Molecular_Biology/PCR/index.html). All of these various tools and protocols can be used in connection with the amplification reactions described herein.

The amplification reactions may be carried out with a variety of different DNA polymerases (or mixture of DNA polymerases), but are preferably carried out in the presence of one or more thermostable polymerases. Suitable thermostable polymerases include, but are not limited to, *Taq* and *Tth* polymerase (commercially available from Applied Biosystems, an Applera Corporation business). Moreover, like conventional RT-PCR amplification reactions, RT-PCR amplification reactions may be carried out with a variety of different reverse transcriptases (or mixture of reverse transcriptases), although in some embodiments thermostable reverse-transcriptions are preferred. Suitable thermostable reverse transcriptases include, but are not limited to, reverse transcriptases such as AMV reverse transcriptase, MuLV, and *Tth* reverse transcriptase.

In some embodiments, the polymerase is selected from the group consisting of Taq DNA polymerase, Taql DNA polymerase, Tbr DNA polymerase, Tfl DNA polymerase, Tru DNA polymerase, Tli DNA polymerase, Tac DNA polymerase, Tne DNA polymerase, Tma DNA polymerase, rTth DNA polymerase, Pfu DNA polymerase, Pho DNA polymerase, Pwo DNA polymerase, Kod DNA polymerase, Bst DNA polymerase, Sac DNA polymerase, Sso DNA polymerase, Poc DNA polymerase, Pab DNA polymerase, Mth DNA polymerase, ES4 DNA polymerase, VENT™ DNA polymerase (New England Biolabs), DEEPVENT™ DNA polymerase (New England Biolabs), PFUTurbo™ DNA polymerase (Stratagene), AmpliTaq® DNA polymerase (Applied Biosystems, an Applera Corporation business), Tth DNA polymerase, T4 DNA polymerase, T5 DNA polymerase, AmpliTaq Gold DNA polymerase (Applied Biosystems, an Applera Corporation business), KlenTaq-1 polymerse, E. Coli DNA polymerase I, Klenow fragment of E. Coli DNA polymerase I, SEQUENASE™ 1.0 DNA polymerase (Amersham Biosciences), SEQUENASE 2.0 DNA polymerase, and mixtures thereof.

Temperatures suitable for carrying out the various denaturation, annealing and primer extension reactions with the polymerases and reverse transcriptases are well-known in the art and/or can be determined empirically.

Optional reagents commonly employed in conventional PCR and RT-PCR amplification reactions, such as reagents designed to enhance PCR, modify $T_m$, or reduce primer-dimer formation, may also be employed in the amplification reactions (see e.g., Patent Nos. 6,410,231; 6,482,588; 6,485,903; and 6,485,944, all of which are incorporated herein by reference). In certain embodiments, the amplifications may be carried out with commercially-available amplification reagents, such as, for example, AmpliTaq® Gold PCR Master Mix, TaqMan® Universal Master Mix and TaqMan® Universal Master Mix No AmpErase® UNG, all of which are available commercially from Applied Biosystems, an Applera Corporation business.

Generally, each amplification primer must be sufficiently long to prime the template-directed synthesis of the sequence of interest under the conditions of the amplification reaction. The exact lengths of the primers may depend on many factors, including but not limited to, the desired hybridization temperature between the primers and template polynucleotide and the complexity of the target polynucleotide sequence to be amplified. The ability to select lengths and sequences of primers suitable for particular applications is within the capabilities of ordinarily skilled artisans. In some embodiments, the primers may contain from about 15 to about 35 nucleotides, although the primers may contain more or fewer nucleotides. Short primer molecules generally require lower temperatures to form sufficiently stable hybrid complexes with the template. In some embodiments, the amplification primers should be designed to have a $T_m$, in relation to primer binding regions in the target polynucleotide, in the range of about 55-75°C. Melting temperatures in this range will tend to insure that the primers remain annealed or hybridized to the target polynucleotide at the initiation of primer extension. The actual temperature used for the primer extension reaction may depend upon, among other factors, the concentration of the primers which are used in the assays. For amplifications carried out with a thermostable polymerase such as *Taq* DNA polymerase, the amplification primers can be designed to have a $T_m$ in the range of about 60 to about 78°C. The melting temperatures of the forward and reverse amplification primers can be different; however, preferably they should be approximately the same.

In some embodiments, the sequences of amplification primers are designed to be substantially complementary to regions of the target polynucleotide that flank the sequence of interest to be amplified. By "substantially complementary" is meant that the sequences of the primers include enough complementarity to hybridize to the target polynucleotide under the temperature and conditions employed in the amplification reaction. In some embodiments, the sequences of amplification primers are designed to be perfectly complementary to regions of the target polynucleotide that flank the sequence of interest to be amplified.

Although in many instances the sequences of the primers may be completely complementary to the template polynucleotide, in some instances it may be desirable to include regions of mis-match or non-complementarity, as is well known in the art. As a specific example, a region of non-complementarity may be included at the 5'-end of one or more of the primers, with the remainder of the primer sequences being completely complementary to their respective target polynucleotide sequences. As another specific example, non-complementary bases or longer regions of non-complementarity can be interspersed throughout the primer, provided that the primer has sufficient complementarity to hybridize to the target polynucleotide sequence under the temperatures and reaction conditions used for the amplification. When primers contain such non-complementary regions, a dummy target polynucleotide as described herein may be designed in accordance with the stability parameters as described above for use in the present methods.

One or more of the amplification primers can include a label, e.g., at the 5' terminus. The term "label" refers to any moiety that, when attached to compounds such as polynucleotides, render such compounds detectable using known detection means, e.g., spectroscopic, photochemical, radioactive, biochemical, immunochemical, enzymatic or chemical means. Exemplary labels include but are not limited to fluorophores, chromophores, radioisotopes, spin labels, enzyme labels, infrared labels, and chemiluminescent labels. Non-limiting examples of a suitable fluorophore comprises a xanthene dye, a fluorescein dye, a rhodamine dye, a cyanine dye, a phthalocyanine dye, a squaraine dye and a bodipy dye. Other examples of labels include members of conventional binding pairs, such as biotin, which may be used with avidin in a capture method or for further concentrating the sample. Another example comprises digoxigenin which may be used with an anti-digoxigenin antibody. Patents teaching the use of such labels include U.S. Patent Nos. 3,817,837; 3,850,752; 3,939,350; 3,996,345; 4,277,437; 4,275,149; and 4,366,241. Means of detecting such labels are well known to those of skill in the art. Amplification primers can also include a sequence that is cleavable such as by a restriction endonuclease (e.g., XhoI).

In some embodiments, primers can also include a 5' "tail" (see, e.g., Bengra et al., 2002, Clin. Chem. 48: 2131-2140 and Myakishev et al., 2001, Genome Res. 11:163-169) for universal amplification, detection and/or purification. In some embodiments, primers can include a tag portion for binding to a tag-complement portion of a mobility modifier (see, e.g., U.S. Patent No. 6,395,486 to Grossman). Exemplary tags and/or tag complements include, but are not limited to, antibodies and associated antigen or hapten, receptors and associated ligands, avidin (or streptavidin) and biotin, and polynucleotide sequences and their complementary sequences. A mobility modifier typically also comprises a tail portion, such as a polymer, for effecting a particular mobility in a mobility-dependent analysis technique.

The chemical composition of the amplification primers is not critical to the success of the amplifications described herein. The only requirement is that the DNA polymerase used in the amplification reaction be able to extend the primers when hybridized to a target polynucleotide. A variety of oligonucleotides that are capable of being extended by DNA polymerases in template-dependent primer extension reactions are known in the art. Examples of these oligonucleotides include, but are not limited to, DNA, RNA, PNA and LNA oligonucleotides, or various combinations and/or chimeras thereof. For example, a chimeric oligonucleotide can comprise a region of DNA fused to a region of, for example, RNA, PNA or LNA. As a specific example, a chimeric amplification primer may include two regions of PNA linked by a 2'-deoxyribonucleotide or region of DNA (see, e.g., International publication No. WO/9640709, incorporated herein by reference, for methods and compositions related to PNA/DNA chimera preparation). The amplification primers may be wholly composed of the standard gene-encoding nucleobases (e.g., cytidine, adenine, guanine, thymine and uracil) or, alternatively, they may include modified nucleobases known by skilled artisans to form base-pairs with the standard nucleobases and to be extendible by polymerases when included in primers. Specific examples of such modified nucleobases include, but are not limited to, 7-deazaguanine and 7-deazaadenine. Other suitable modified or non-standard nucleobases will be apparent to those of skill in the art.

In addition, the amplification primers may include one or more modified interlinkages, such as one or more phosphorothioate or phosphorodithioate interlinkages, as is well-known in the art.

All of these types of various oligonucleotides, or mixtures of such oligonucleotides, may be used as amplification primers in the various amplifications described herein. In one embodiment, the primers used in the amplification reaction are DNA oligonucleotides.

Methods of analyzing amplified nucleic acid are well known in the art and fully described in the literature. The original amplification method is the polymerase chain reaction described by Mullis, et al. as described above.. A wide array of strategies for amplification have been described and include LCR and RCA (see, e.g., U.S. Patent Nos. 4,683,195, 4,683,302, 5,130,238, 5,354,668, 5,427,930, and 5,455,166). The particular method used is not a critical part of the present invention. One of skill in the art can select a suitable analysis method depending on the application.

Various end-point analyses can be used, as mentioned above. Quantitative or real-time PCR reactions, as well as different types of reagents and/or labeled oligonucleotide probes useful for monitoring the amplification in real time, can also be used. One exemplary assay which utilizes the 5'-exonuclease assay to monitor the amplification as a function of time is referred to as the 5'-exonuclease gene quantification assay (see, e.g., U.S. Patent Nos. 5,210,015 and 5,538,848 and Lie & Petropoulos, 1998, Curr. Opin. Biotechnol. 14:303-308). Another assay utilizes intercalating or other dyes to monitor an amplification as a function of time (see, e.g., U.S. Patent No. 5,994,056).

Studies have shown that initial copy number can be quantified during real-time PCR analysis based on threshold cycle (Ct) (see, e.g., Higuchi, R., et al., 1993, Biotechnology 11:1026-1030). Ct is defined as the cycle at which fluorescence is determined to be statistically significant above background. The threshold cycle is inversely proportional to the log of the initial copy number. The more template that is present to begin with, the fewer the number of cycles it takes to get to a point where the fluorescent signal is detectable above background.

An amplification as described herein can be effectively carried out in the presence of oligonucleotide probes, such as, for example, non-priming oligonucleotide probes designed for quantitative or real-time PCR analysis. Such probes can be used to monitor the amplification of the target polynucleotide and/or the dummy target polynucleotide. In some embodiments, oligonucleotide probes present in an amplification are suitable for monitoring the amount of amplicon (s) produced as a function of time. Such oligonucleotide probes include, but are not limited to, the 5'-exonuclease assay (TaqMan®) probes described above (see also U.S. Patent No. 5,538,848), various stem-loop molecular beacons (see, e.g., U.S. Patent Nos. 6,103,476 and 5,925,517 and Tyagi & Kramer, 1996, Nature Biotechnology 14:303-308), stemless or linear beacons (see, e.g., WO 99/21881), PNA Molecular Beacons™ (see, e.g., U.S. Patent Nos. 6,355,421 and 6,593,091), linear PNA beacons (see, e.g., Kubista et al., 2001, SPIE 4264:53-58), non-FRET probes (see, e.g., U.S. Patent No. 6,150,097), Sunrise®/Amplifluor® probes (U.S. Patent No. 6,548,250), stem-loop and duplex Scorpion™ probes (Solinas et al., 2001, Nucleic Acids res. 29:E96 and U.S. Patent No. 6,589,743), bulge loop probes (U.S. Patent No. 6,590,091), pseudo knot probes (U.S. Patent No. 6,548,250), cyclicons (U.S. Patent No. 6,383,752), MGB Eclipse™ probe (Epoch Biosciences), hairpin probes (U.S. Patent No. 6,596,490), peptide nucleic acid (PNA) light-up probes, self-assembled nanoparticle probes, and ferrocene-modified probes described, for example, in U.S. Patent No. 6,485,901; Mhlanga et al., 2001, Methods 25:463-471; Whitcombe et al., 1999, Nat. Biotechnol. 17:804-807; Isacsson et al., 2000, Mol. Cell. Probes. 14:321-328; Svanvik et al., 2000, Anal Biochem. 281:26-35; Wolffs et al., 2001, Biotechniques 766:769-771; Tsourkas et al., 2002, Nucleic Acids Res. 30:4208-4215; Riccelli et al., 2002, Nucleic Acids Res. 30:4088-4093; Zhang et al., 2002, Shanghai. 34:329-332; Maxwell et al., 2002, J. Am. Chem. Soc. 124:9606-9612; Broude et al., 2002, Trends Biotechnol. 20:249-56; Huang et al., 2002, Chem. Res. Toxicol. 15:118-126; and Yu et al., 2001, J. Am. Chem. Soc. 14:11155-11161, all of which are incorporated herein by reference.

Oligonucleotide probes that can be present in the amplification are not limited to 5'-exonuclease probes. In some embodiments, any labeled or unlabeled single-stranded oligonucleotide which is complementary to all or part of an amplified target sequence (e.g., region 22), may be present in the amplification. In some embodiments, any labeled or unlabeled single-stranded oligonucleotide which is complementary to all or part of an intermediate region of a dummy target polynucleotide (e.g., region 38), may be present in the amplification.

Like the primers discussed above, such oligonucleotide probes may be DNA, RNA, PNA, LNA or chimeras composed of one or more combinations thereof. The oligonucleotides may be composed of standard or non-standard nucleobases or mixtures thereof and may include one or more modified interlinkages, as previously described in connection with the amplification primers.

In some embodiments, each oligonucleotide probe is complementary to at least a region of a specified amplicon. A probe can be completely complementary to the amplicon, or may be substantially complementary thereto (at least about 65% complementary over a stretch of at least 15 to 75 nucleotides). In other embodiments, the probes are at least about 75%, 85%, 90%, or 95% complementary to the amplicon (see, e.g., Kanehisa, M., 1984, Nucleic Acids Res. 12: 203, incorporated herein by reference). The exact degree of complementarity between a specified oligonucleotide probe and an amplicon will depend upon the desired application for the probe and will be apparent to those of skill in the art.

The lengths of an oligonucleotide probe can vary broadly, and in some embodiments can range from as few as two as many as tens or hundreds of nucleotides, depending upon the particular application for which the probe was designed. In some embodiments, an oligonucleotide probe can range in length from about 15 to 35 nucleotides. In some embodiments, an oligonucleotide probe can range in length from about 15 to 25 nucleotides. In some embodiments, an oligonucleotide probe can range from 25 to 75 nucleotides. In some embodiments, a probe can range in length from about 6 to 75 nucleotides or from about 12 to 22 nucleotides. An oligonucleotide probe can include a 5' tag portion for binding with a mobility modifier (e.g., as described in U.S. Patent No. 6,395,486).

Oligonucleotide probes can be designed for monitoring the accumulation of the target polynucleotide and/or the dummy target polynucleotide. Ct values for the target polynucleotide and/or the dummy target polynucleotide can be determined. When both the target polynucleotide and dummy target polynucleotide are monitored, probes having distinguishable labels can be used. In some embodiments of the methods herein, the Ct value for the dummy target polynucleotide is greater than for the target polynucleotide. In some embodiments, the Ct value for the amplification of the dummy target polynucleotide is at least about 3 cycles greater than the Ct value for the amplification of the target polynucleotide. In some embodiments, the Ct value for the amplification of the dummy target polynucleotide is at least about 1, 2, 3, 4, 5, 6, 7, 8, 9, or 10 cycles greater than the Ct value for the amplification of the target polynucleotide.

Multiplex amplifications have been described (see, e.g, U.S. Patent No. 6,605,451) and employ a plurality of primer pairs to amplify various target sequences simultaneously. A plurality of dummy target polynucleotides, each designed for use with a different primer pair in a multiplex amplification, can be use as described herein.

An amplification may also be carried out in the presence of dye molecules suitable for, for example, monitoring the accumulation of amplification products at the end of the amplification and/or during the amplification as a function of time. In one embodiment, such dyes include dyes that produce a detectable signal, such a fluorescence, when bound to double-stranded polynucleotides. Non-limiting examples of suitable dyes include common nucleic acid stains, such as intercalating dyes and minor groove binding dyes, as are well-known in the art. In a specific embodiment, the dye is SYBR® Green I or II, ethidium bromide, or YO-PRO-1 (available from Molecular Probes, Eugene, OR). Such dyes can be used at concentrations commonly employed in the art (see, e.g., U.S. Patent No. 5,994,056).

The present methods, compositions, and kits are compatible with other methods, compositions, and kits for reducing non-specific amplification. For example, the present invention can be used in an amplification carried out using a reversibly inactivated polymerase enzyme as described in U.S. Patent Nos. 5,677,152, and 5,773,258, each incorporated herein by reference. The use of a reversibly inactivated enzyme, which is re-activated under the high temperature reaction conditions, further reduces non-specific amplification by inhibiting primer extension of any deblocked primers prior to the start of the reaction. A reversibly inactivated thermostable DNA polymerase, developed and manufactured by Hoffmann-La Roche (Nutley, N.J.) and marketed by Perkin Elmer (Norwalk, Conn.), is described in Birch et al., 1996, Nature 381:445-446, incorporated herein by reference. Reversibly blocked amplification primers can also be used (e.g., see U.S. Patent No. 6,509,157).

Also provided herein are reagents and kits suitable for carrying out an amplification as described herein. Such kits can include reagents suitable for carrying out conventional PCR and RT-PCR amplification reactions. In some embodiments, the kit comprises forward and reverse amplification primers suitable for amplifying a target polynucleotide of interest and a dummy target polynucleotide, wherein the dummy target polynucleotide comprises: a first dummy strand comprising a 5'-region, the complement of which is capable of hybridizing to the forward amplification primer; a 3'-region capable of hybridizing to the reverse amplification primer; and an optional intermediate region disposed between the 5'- and 3'- regions; and an optional second dummy strand complementary to the first dummy strand. A kit can comprise at least one DNA polymerase. A kit can include dNTPs, and/or buffer, suitable for carrying out template-dependent primer extension. In some embodiments, at least one of the amplification primers comprises a capture moiety, or is labeled with a detectable moiety. In some embodiments, a kit can include a polynucleotide binding agent that produces a detectable signal proportional to the amount of amplification product (e.g., SYBR Green 1). In some embodiments, a kit can include a sequence specific probe wherein said probe is labeled with a labeling system suitable for monitoring amplification of a target polynucleotide as a function of time. In some embodiments, a kit can include a sequence specific probe wherein said probe is labeled with a labeling system suitable for monitoring amplification of a dummy target polynucleotide as a function of time. A kit can include written directions for carrying out methods as described herein. In some embodiments, a kit can include detection means for detecting amplification products. A kit can include a dummy target polynucleotide comprising a first strand having the formula $(UP_1)-(B)_n-(UP_2)$ as described herein.

Unless defined otherwise, all technical and scientific terms used herein have the same meaning as commonly understood by one of ordinary skill in the art to which this disclosure belongs. Unless mentioned otherwise the techniques employed or contemplated herein are standard methodologies well known to one of ordinary skill in the art. The materials, methods and examples are illustrative only and not limiting.

All numerical ranges in this specification are intended to be inclusive of their upper and lower limits.

Other features of the disclosure will be come apparent in the course of the following descriptions of some embodiments which are given for illustration of the disclosure and are not intended to be limiting thereof.

**EXAMPLES**

**1.3 Example 1 Effect of Dummy target polynucleotide on the level**

**of Amplification Artifacts in a PCR Amplification**

This example shows the effect of the presence of a dummy target polynucleotide on the level of amplification artifacts generated during a PCR amplification.

The primers and dummy target polynucleotides were prepared by automated synthesis (Model 394 DNA/RNA synthesizer, Applied Biosystems, an Applera Corporation business) according to general procedures described in the user manual. The synthesized primers and dummy target polynucleotides were desalted and dissolved in 1X TE (10 mM Tris-8.0, 1 mM EDTA).

The PCR primers employed were:

Forward primer: GCTTGCCTGCTCGACTTAGA (SEQ I D NO:1)
Reverse primer: CCACGTCGCTATCCAGTGAT (SEQ I D NO:2)

The target polynucleotide was a double stranded DNA fragment in which the plus strand had the sequence:

TTGCCTGCTCGACTTAGATCTGGCAACACTATGGGTACGCGGGCGTTCAGATTTTCCTGCTAAT ATCTCATTTTCTCTTGTCATCACTGGATAGCGACGTGG (SEQ ID NO:3)

The dummy target polynucleotides used were:

TTGCCTGCTCGACTTAGA*TTTTTTTTTTTTTGGGGGGGGGGGGGG*ATCACTGGA**C**AGCGACGT (SEQ ID NO:4)

TTGCCTG**G**TCGACTTAGA*TTTTTTTTTTTTTGGGGGGGGGGGGGG*ATCACTGGA**C**AGCGACGT (SEQ ID NO:5)

TTGC**G**TGCTCGACTTAGA*TTTTTTTTTTTTTGGGGGGGGGGGGGG*ATCACTGGATAGC**C**ACGT (SEQ ID NO:6)

TTGC**G**TGCTCGACTTAGA*TTTTTTTTTTTTTGGGGGGGGGGGGGGATCAC*TGGA**C**AGC**C**ACGT (SEQ ID NO:7)

For each dummy target polynucleotide, the intermediate region is indicated by italicized nucleotide letters. The 5' region and the 3' region flank the intermediate region on the left and right sides, respectively. The underlined nucleotides indicate a site of mismatch in relation to the corresponding primer sequence.

PCR amplification was carried out in an amplification solution (10 $\mu$l) comprising 5 $\mu$l of AmpliTaq Gold® PCR Master Mix (Part no. 4324020, Applied Biosystems, an Applera Corporation business), 5 $\mu$l of a solution containing the target polynucleotide (0.01 - 10 pM), and 1 $\mu$M each (final concentration) of forward PCR primer and reverse PCR primers (as indicated below). The dummy target polynucleotide, when included, was present at a final concentration in the range of 0.01 - 10 pM. In certain amplifications, the dummy target polynucleotide and/or the target polynucleotide were not included.

The AmpliTaq Gold PCR Master Mix is supplied as a 2X proprietary mixture comprising buffer, dNTPs, and AmpliTaq Gold DNA polymerase , and was used in accordance with the instructions provided by the manufacturer.

The PCR samples were thermal cycled to conduct PCR in a thermal cycler (GeneAmp® PCR System 9700, Applied Biosystems, an Applera Corporation business) according to the following programs: (a) 10 minute hold at 95°C, (b) 35 cycles of 2 seconds at 92°C, then 30 seconds at 60°C, followed by 72°C for 30 seconds. The samples were then held at 4°C.

Amplification products were analyzed by denaturing gel electrophoresis using 15% polyacrylamide gels, followed by application of SYBR Green I stain.

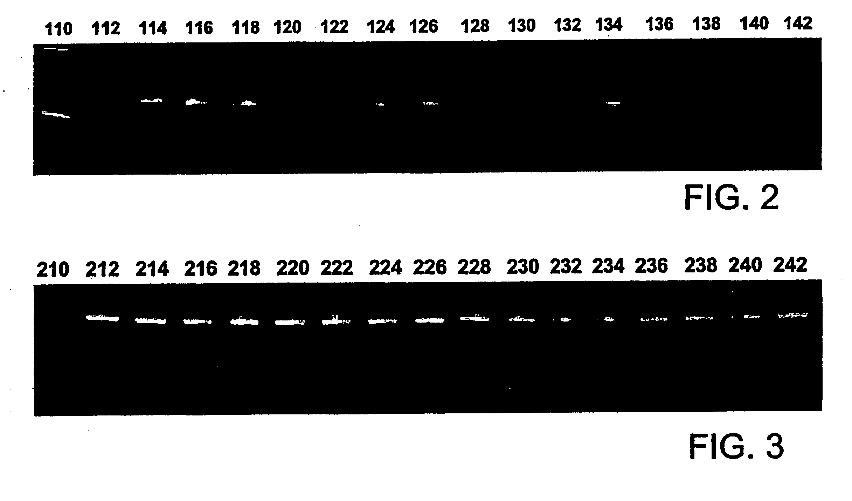

A series of amplifications were conducted in the presence of PCR primers (SEQ ID NOs: I and 2) and in the presence of various dummy target polynucleotides, and the amplification products were analyzed by electrophoresis (15% acrylamide gel) as shown in FIG. 2. Lane 110 is a 25 base pair (bp) ladder (Invitrogen, catalog no. 10597-001). The other samples loaded onto the gel (approximately 250 ng DNA per lane) were from PCR amplifications that included dummy target polynucleotides: lanes 112, 114, 116, 118 (SEQ ID NO:4); lanes 120, 122, 124, 126 (SEQ ID NO:5); lanes 128, 130, 132, 134 (SEQ ID NO:6); and lanes 136, 138, 140, 142 (SEQ ID NO:7). In each set of four lanes, from left to right, the sample was prepared from PCR in which the dummy target polynucleotide was present in the amplification reaction at a concentration of 0.01, 0.1, 1.0 or 10 pM, respectively.

The band at about 60 bp is due to amplification of the dummy target polynucleotide. As shown, the dummy target polynucleotide having only a single mismatch (SEQ ID NO:4) when paired with its PCR primers yielded detectable amount of product at all concentration tested.

The other dummy target polynucleotides, having two or more mismatches yielded lower amounts of amplification product, due to lower affinity for the primers. This trend is in agreement with the calculated stability (as reflected in Tm and free energy of duplex formation) of hybrids between the dummy target polynucleotide and the primers, as described hereinbelow. For example, the target polynucleotide having the lowest Tm against the primers (SEQ ID NO:7) gave the lowest amount of PCR product (FIG. 2, lanes 136, 138, 140 and 142). SEQ ID NO: 4 was selected for use in further experiments because it provided a single, and detectable, band at all of the concentrations tested.

FIG. 3 shows the results of polyacrylamide gel electrophoretic analysis of amplification reactions conducted as described in FIG. 2 except that target polynucleotide (SEQ ID NO:3) (about 100-300 ng) was present during the amplification in each reaction. SEQ ID NO:3 was the product of an OLA, as further described below. The PCR amplifications included dummy target polynucleotides: lanes 212, 214, 216, 218, SEQ ID NO:4; lanes 220,222,224,226, SEQ ID NO:5; lanes 228,230,232,234, SEQ ID NO:6; and lanes 236, 238, 240, 242, SEQ ID NO:7. In each set of four lanes, the sample was prepared from PCR in which the dummy target polynucleotide was present in the reaction at a concentration of 0.01, 0.1, 1.0 or 10 pM, respectively. In the presence of target polynucleotide, the amplification of the dummy target polynucleotide was suppressed, as compared to the levels observed in FIG. 2. This is an example of a titration procedure for ensuring that the amplification of the dummy target polynucleotide does not out-compete, or significantly interfere, with the amplification of the target polynucleotide.

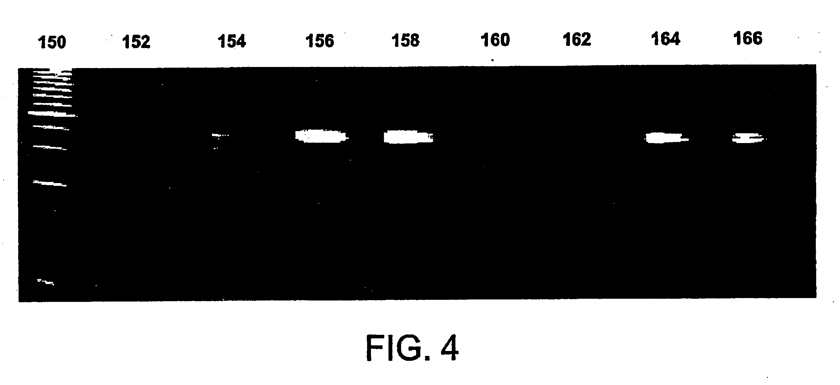

Another series of amplifications were performed in the presence of PCR primers (SEQ ID NOs: 1 and 2) to determine the effect of the presence of the dummy target polynucleotide on the appearance of primer-induced artifacts (FIG. 4). Lane 150 is a 25 bp ladder. The other samples loaded onto the gel were from PCR amplifications of OLA reaction products: lanes 152, 154 were from PCR amplifications of OLA reactions which lacked genomic template (i.e., "no template controls") and in which the PCR was conducted in the absence of dummy target polynucleotide; lanes 156, 158 were from PCR amplification of OLA products (approximately 5-50 fM of the OLA product identified by SEQ 10 NO: 3) and in which the PCR was conducted in the absence of dummy target polynucleotide; lanes 160, 162 were from PCR amplifications as described for lanes 152 and 154, respectively, but conducted in the presence of dummy target polynucleotide (SEQ ID NO:4) (about 0.1 pM); and lanes 164, 166 were from PCR amplification as described for lanes 156 and 158, respectively, but conducted in the presence of dummy target polynucleotide (SEQ ID NO:4) (about 0.1 pM). In the OLA reactions analyzed in FIG. 4, the OLA product (SEQ ID NO:3) was formed by the ligation of two oligonucleotide probes which had been hybridized adjacently to a genomic target. After ligation, the reaction was subjected to exonuclease digestion in order to digest the remaining single stranded ligation probes. However, Applicants have routinely found that this digestion is incomplete, and that some residual amount of the probes remains. The same genomic template (obtained from a test subject) was used in preparing the OLA product analyzed in lanes 156 and 164, and another genomic template was used in preparing the OLA product analyzed in lanes 158 and 166.

The results in FIG. 4 demonstrate that in PCR of OLA conducted in the absence of dummy target polynucleotide and in the absence of genomic template (i.e., "no template controls"), significant amounts of amplification-induced artifacts were observed (lanes 152 and 154). Such artifacts can interfere with the interpretation of a "yes-no" test such as OLA since they generate a significant background signal even in the absence of template. Sequencing of these artifacts (data not shown) confirmed that they resulted from nonspecific priming between PCR primers and ligation probes used in the OLA. When genomic template was present during the OLA, the expected target polynucleotide amplification product (SEQ ID NO:3) was observed after PCR (lanes 156 and 158). In amplifications that included a dummy target polynucleotide, amplification-induced artifacts were markedly reduced, especially for the "no template control" reactions (compare lanes 152 with 160, and 154 with 162), thus significantly improving the accuracy of the OLA.

1.4 **Example 2 Calculation of Melting Temperature and Free**

**Energy of Duplex Formation**

The following table summarizes calculated melting temperature and free energy parameters for the binding of primers, SEQ ID NO: 1 (at the 5' region) and SEQ ID NO:2 (at the 3' region), to the indicated dummy target polynucleotides (SEQ ID Nos: 4 - 7).

| Line | SEQ ID NO. | 5' region $\Delta Tm$ (°C) | 5' region $\Delta\Delta G$ (kcal/mol) | 3'region $\Delta Tm$ (°C) | 3'region $\Delta\Delta G$ (kcal/mol) |
|------|------------|-----------|-------------|-----------|-------------|
| A | 4 | | | -4.5 | 2.2 |
| B | 5 | -4.6 | 2.0 | -4.5 | 2.2 |
| C | 6 | -6.3 | 2.6 | -10.7 | 4.4 |
| D | 7 | -6.3 | 2.6 | -18.5 | 6.7 |

Tm and free energy values were obtained using an algorithm utilizing a nearest-neighbor model and parameters as described by SantaLucia (Proc. Natl. Acad. Sci. (1998) 95:1460-1465), and assuming a cation salt concentration of 0.1 M.

For the 5' region and the 3' region, Tm values, and free energy values, were calculated for primer binding regions in dummy target polynucleotide sequences that were fully complementary to the primer sequences, and also for the dummy target polynucleotide sequences (as shown in the above table) having one or more mistmaches with the primers. The resulting change in these Tm values (and free energy values) are shown in the Table. For each binding region, the presence of a single mismatch lowered the Tm value (and increased the free energy value) reflecting the lower stability of the hybrid. For example, in Line A, the presence of a single mismatch lowered the Tm by 4.5°C and increased the free energy value by 2.2 kcal/mole. An increase in the number of mismatches further lowered the Tm value (and further increased the free energy value), reflecting the further decrease in stability of the hybrid when mismatches were present. For example, considering the 3'-region, the presence of two mismatches (line D) yielded a decrease in Tm (and an increase in $\Delta\Delta G$ value) as compared to the presence of a single mismatch (Line C). The Table also illustrates that the position of the mismatch influences the observed changes in Tm and $\Delta\Delta G$. For example, considering the 3'-region, the presence of a single mismatch, but at a different position, gave differing results (Lines B and C).

Calculated Tm and $\Delta G$ values for the binding of primers (SEQ ID NO:1 and SEQ ID NO:2) to the target polynucleotide SEQ ID NO:3 were as follows:

SEQ ID NO:1    Tm=65.2    $\Delta G$=-16.4
SEQ ID NO:2    Tm=64.8    $\Delta G$=-16.8

The primers were fully complementary to their respective binding sites on the target polynucleotide.

While the foregoing has presented some embodiments of the present invention, it is to be understood that these embodiments have been presented by way of example only. It is expected that others will perceive and practice variations which, though differing from the foregoing do not depart form the spirit and scope of the invention as described and claimed herein. All patent applications, patents, and literature references cited in this specification are hereby incorporated by reference in their entirety. In this application, the use of the singular comprises the plural unless specifically stated otherwise. "Comprise" and "comprises" are not intended to be limiting.

**Claims**

1. A method of amplifying a polynucleotide sequence of interest, comprising: amplifying a target polynucleotide in the presence of a polymerase, a forward amplification primer, a reverse amplification primer, dNTPs suitable for template-dependent primer extension and a dummy target polynucleotide, wherein the target polynucleotide comprises:

   (i) a first target strand comprising a 5'-region, the complement of which is capable of hybridizing to the forward amplification primer; a 3'-region capable of hybridizing to the reverse amplification primer; and a sequence of interest disposed between the 5'-and 3'regions; and
   (ii) an optional second target strand that is complementary to the first target strand, and the dummy target polynucleotide comprises:

   (i) a first dummy strand comprising a 5'-region, the complement of which is capable of hybridizing to the forward amplification primer; a 3'-region capable of hybridizing to the reverse amplification primer; and an optional intermediate region disposed between the 5' and 3'-regions; and

(ii) an optional second dummy strand complementary to the first dummy strand, wherein the $\Delta G$ of a hybrid formed between the forward amplification primer and the optional second dummy strand is at least 1. 5 kcal/mol greater than the $\Delta G$ of a hybrid formed between the forward amplification primer and the optional second target strand and/or the $\Delta G$ of a hybrid formed between the reverse amplification primer and the first dummy strand is at least 1. 5 kcal/mol greater than the $\Delta G$ of a hybrid formed between the reverse amplification primer and the first target strand.

the method beings carried out in the presence of a plurality of target polynucleotides, each of which comprises:

(i) a first target strand comprising a 5'-region, the sequence of which is identical to the universal forward priming sequence; a 3'-region, the sequence of which is complementary to the universal reverse priming sequence; and a sequence of interest disposed between the 3'- and 5'-regions, wherein each of the plurality of target polynucleotides has a unique sequence of interest; and
(ii) an optional second target strand complementary to the first target strand.

2. The method of Claim 1 in which the sequence of the dummy target polynucleotide is such that the hybrid formed between the first dummy strand and the reverse amplification primer comprises at least one mismatch and/or the hybrid formed between the optional second dummy strand and the forward amplification primer comprises at least one mismatch.

3. The method of Claim 1 in which the $T_m$ of the hybrid formed between the forward amplification primer and the optional second target strand is at least 2°C greater than the $T_m$ of the hybrid formed between the forward amplification primer and the optional second dummy strand under the conditions of the amplifying and/or the $T_m$ of the hybrid formed between the reverse amplification primer and the first target strand is at least 2°C greater than the $T_m$ of the hybrid formed between the reverse amplification primer and the first dummy strand under the conditions of the amplifying.

4. The method of Claim 1 in which the dummy target polynucleotide has a sequence, and is present at a concentration, such that the level of amplification artifacts is lower when the amplifying is carried out in the presence of the dummy target polynucleotide than in its absence or
in which the dummy target polynucleotide has a sequence, and is present at a concentration, such that after about 20 amplification cycles the amount of target polynucleotide amplicon generated is approximately 10-fold greater than the amount dummy target polynucleotide amplicon generated.

5. The method of Claim 1 in which the 3'-region of the first dummy strand comprises a modified nucleobase or/and in which the optional second dummy strand comprises a modified nucleobase in the region that hybridizes to the forward amplification primer.

6. The method of Claim I in which the 3'-region of the first dummy strand comprises at least one modified internucleotide linkage.

7. The method of Claim 1 in which the optional second dummy strand comprises at least one modified internucleotide linkage in the region that hybridizes to the forward amplification primer.

8. The method of Claim 1 in which the forward amplification primer comprises a universal forward amplification priming sequence and the reverse amplification primer comprises a universal reverse amplification priming sequence.

9. The method of Claim 1 in which at least one of the amplification primers comprises a cleavage site for a restriction enzyme.

10. The method of Claim 1 in which the forward amplification primer further comprises a sequence-specific capture sequence or/and
in which the reverse amplification primer further comprises a capture moiety.

11. The method of Claim 1 in which the cycle threshold (Ct) value for the amplification of the dummy target polynucleotide is at least 3 cycles greater than the cycle threshold value for the amplification of the target polynucleotide.

**12.** The method of Claim 1 in which at least one of the amplification primers is labeled with a detectable label.

**13.** The method of Claim 1 in which the amplifying is carried out in the presence of a polynucleotide binding agent that produces a detectable signal proportional to the amount of amplification product generated or in which the amplifying is carried out in the presence of at least one of (i) a first sequence specific probe suitable for monitoring the accumulation of amplification product from the target polynucleotide as a function of time and (ii) a second sequence specific probe for monitoring the amount accumulation of amplification product from the dummy target polynucleotide as a function of time.

**14.** A kit for use in amplifying a target polynucleotide sequence, comprising: a dummy target polynucleotide suitable for use in an amplification reaction comprising a target polynucleotide of interest, forward and reverse amplification primers suitable for amplifying said target polynucleotide of interest and a dummy target polynucleotide, wherein the dummy target polynucleotide comprises: a first dummy strand comprising a 5'-region, the complement of which is capable of hybridizing to the forward amplification primer; a 3'-region capable of hybridizing to the reverse amplification primer; and an optional intermediate region disposed between the 5'-and 3'-regions; and an optional second dummy strand complementary to the first dummy strand; wherein the ΔG of a hybrid formed between the forward amplification primer and the optional second dummy strand is at least 1.5 kcal/mol greater than the ΔG of a hybrid formed between the forward amplification primer and the optional second target strand and/or the ΔG of a hybrid formed between the reverse amplification primer and the first dummy strand is at least 1. 5 kcal/mol greater than the ΔG of a hybrid formed between the reverse amplification primer and the first target strand.

**15.** A polynucleotide comprising a first strand having the formula: $(UP_1)-(B)_n-(UP_2)$ wherein:

UP$_1$, represents a sequence, the complement of which is capable of hybridizing to a universal priming sequence;
UP$_2$ represents a sequence capable of hybridizing to a universal priming sequence;
each B, independently of the others, represents a nucleotide selected from A, C, T, G, I and U; and
n represents an integer from 0 to 1000, and further wherein the sequence of UP$_1$ is such that a hybrid formed between the complement of UP$_1$ and a universal priming sequence comprises at least one mismatch and/or the sequence of UP$_2$ is such that a hybrid formed between UP$_2$ and a universal priming sequence comprises at least one mismatch.

FIG.1

110 112 114 116 118 120 122 124 126 128 130 132 134 136 138 140 142

**FIG. 2**

210 212 214 216 218 220 222 224 226 228 230 232 234 236 238 240 242

**FIG. 3**

FIG. 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4683195 A, Mullis
- US 4683202 A, Mullis
- US 4965188 A, Mullis
- US 5229297 A
- US 5176995 A
- US 5310652 A
- US 5322770 A
- US 6297016 B
- US 6025139 A
- US 4800159 A
- US 5561058 A
- US 5618703 A
- US 5693517 A
- US 5876978 A
- US 6087098 A
- US 6436677 A
- US 6485917 A
- US 3817837 A
- US 3850752 A
- US 3939350 A
- US 3996345 A
- US 4277437 A
- US 4275149 A
- US 4366241 A
- US 6395486 B, Grossman
- WO 9640709 A
- US 4683302 A
- US 5130238 A
- US 5354668 A
- US 5427930 A
- US 5455166 A
- US 5210015 A
- US 5538848 A
- US 5994056 A
- US 6103476 A
- US 5925517 A
- WO 9921881 A
- US 6355421 B
- US 6593091 B
- US 6150097 A
- US 6548250 B
- US 6589743 B
- US 6590091 B
- US 6383752 B
- US 6596490 B
- US 6485901 B
- US 6605451 B
- US 5677152 A
- US 5773258 A
- US 6509157 B

### Non-patent literature cited in the description

- **Sambrook et al.** Molecular Cloning--A Laboratory Manual. Cold Spring Harbor Laboratory, 1989
- **Ausubel et al.** Current Protocols in Molecular Biology. John Wiley & Sons, 1995
- **SantaLucia.** *Proc. Natl. Acad. Sci. USA,* 1998, vol. 95, 1460-1465
- **Frier et al.** *Proc. Natl. Acad. Sci. USA,* 1986, vol. 83, 9373-9377
- **Breslauer.** *Proc. Natl. Acad, Sci. USA,* 1986, vol. 83, 3746-3750
- **Rychlik et al.** *Nucleic Acids Res.,* 1989, vol. 17, 8543-8551
- **Rychlik et al.** *Nucleic Acids Res.,* 1989, vol. 18, 6409-6412
- **Wetmur.** *Crit. Rev. Biochem. Mol. Biol.,* 1991, vol. 26, 227-259
- **Osborne.** *CABIOS,* 1991, vol. 8, 83
- **Montpetit et al.** *J. Virol. Methods,* 1992, vol. 36, 119-128
- **Eckert et al.** PCR: A Practical Approach. IRL Press, 1991, vol. 1, 225-244
- **Rozen et al.** Bioinformatics Methods and Protocols: Methods in Molecular Biology. Humana Press, 2000, 365-386
- PCR Essential Data. J. W. Wiley & Sons, 1995
- PCR Protocols: A Guide to Methods and Applications. Academic Press, 1990
- **Bengra et al.** *Clin. Chem.,* 2002, vol. 48, 2131-2140
- **Myakishev et al.** *Genome Res.,* 2001, vol. 11, 163-169
- **Lie ; Petropoulos.** *Curr. Opin. Biotechnol.,* 1998, vol. 14, 303-308
- **Higuchi, R. et al.** *Biotechnology,* 1993, vol. 11, 1026-1030
- **Tyagi ; Kramer.** *Nature Biotechnology,* 1996, vol. 14, 303-308
- **Kubista et al.** *SPIE,* 2001, vol. 4264, 53-58

- **Solinas et al.** *Nucleic Acids res.,* 2001, vol. 29, E96
- **Mhlanga et al.** *Methods,* 2001, vol. 25, 463-471
- **Whitcombe et al.** *Nat. Biotechnol.,* 1999, vol. 17, 804-807
- **Isacsson et al.** *Mol. Cell. Probes,* 2000, vol. 14, 321-328
- **Svanvik et al.** *Anal Biochem.,* 2000, vol. 281, 26-35
- **Wolffs et al.** *Biotechniques,* 2001, vol. 766, 769-771
- **Tsourkas et al.** *Nucleic Acids Res.,* 2002, vol. 30, 4208-4215
- **Riccelli et al.** *Nucleic Acids Res.,* 2002, vol. 30, 4088-4093
- **Zhang et al.** *Shanghai,* 2002, vol. 34, 329-332
- **Maxwell et al.** *J. Am. Chem. Soc.,* 2002, vol. 124, 9606-9612
- **Broude et al.** *Trends Biotechnol.,* 2002, vol. 20, 249-56
- **Huang et al.** *Chem. Res. Toxicol.,* 2002, vol. 15, 118-126
- **Yu et al.** *J. Am. Chem. Soc.,* 2001, vol. 14, 11155-11161
- **Kanehisa, M.** *Nucleic Acids Res.,* 1984, vol. 12, 203
- **Birch et al.** *Nature,* 1996, vol. 381, 445-446
- **SantaLucia.** *Proc. Natl. Acad. Sci.,* 1998, vol. 95, 1460-1465

SEQUENCE LISTING

<110>  Applera Corporation

<120>  Methods, Compositions and Kits for Use in Polynucleotide
       Amplification

<130>  110-042T1

<150>  EP 05724335.4 (PCT/US2005/006765)
<151>  2005-03-01

<150>  US 60/549,490
<151>  2004-03-01

<160>  7

<170>  PatentIn version 3.3

<210>  1
<211>  20
<212>  DNA
<213>  Artificial

<220>
<223>  Synthetic DNA

<400>  1
gcttgcctgc tcgacttaga                                                   20


<210>  2
<211>  20
<212>  DNA
<213>  Artificial

<220>
<223>  Synthetic DNA

<400>  2
ccacgtcgct atccagtgat                                                   20


<210>  3
<211>  102
<212>  DNA
<213>  Artificial

<220>
<223>  Synthetic DNA

<400>  3
ttgcctgctc gacttagatc tggcaacact atgggtacgc gggcgttcag attttcctgc      60

taatatctca ttttctcttg tcatcactgg atagcgacgt gg                        102


<210>  4
<211>  60
<212>  DNA
<213>  Artificial

```
<220>
<223>  Synthetic DNA

<400>  4
ttgcctgctc gacttagatt tttttttttt gggggggggg ggatcactgg acagcgacgt      60


<210>  5
<211>  60
<212>  DNA
<213>  Artificial

<220>
<223>  Synthetic DNA

<400>  5
ttgcctggtc gacttagatt tttttttttt gggggggggg ggatcactgg acagcgacgt      60


<210>  6
<211>  60
<212>  DNA
<213>  Artificial

<220>
<223>  Synthetic DNA

<400>  6
ttgcgtgctc gacttagatt tttttttttt gggggggggg ggatcactgg atagccacgt      60


<210>  7
<211>  60
<212>  DNA
<213>  Artificial

<220>
<223>  Synthetic DNA

<400>  7
ttgcgtgctc gacttagatt tttttttttt gggggggggg ggatcactgg acagccacgt      60
```